# EUROPÄISCHE PATENTANMELDUNG

(11) **EP 0 589 324 A2**
(43) Veröffentlichungstag der Anmeldung: **30.03.1994**
(21) Anmeldenummer: 93114686.4
(22) Anmeldetag: 13.09.1993
(51) Int. Cl.: A61B 17/36, A61B 17/39, A61N 1/06

(54) **Neutralelektrode und Verfahren zu deren Wiederverwendung für ein Hochfrequenz-Chirurgiegerät mit Applikationsüberwachungseinrichtung**

(30) Priorität: 25.09.1992 DE 4232255
(71) Anmelder: SIEMENS AKTIENGESELLSCHAFT, D-80333 München (DE)
(72) Erfinder: Weller, Gerhard, Dipl.-Ing. (FH), D-91054 Erlangen (DE)

(57) **Zusammenfassung**

Es wird eine wiederverwendbare, mehrteilige Neutralelektrode (1 bis 4) mit einem einmalverwendbaren, abziehbaren Beschichtungsmittel (12) und einem doppelseitigen Klebefilm (13, 14) vorgeschlagen.

## Beschreibung

Die Erfindung betrifft eine mehrteilige Neutralelektrode für ein Hochfrequenz-Chirurgiegerät, bei der Teilelektroden durch Isolierstreifen getrennt auf einem isolierenden, elastischen Träger angeordnet sind, wobei die Teilelektroden über einen am Träger vorgesehenen elektrischen Leitungsanschluß und ein Elektrodenkabel an das HF-Chirurgiegerät anschließbar sind, das eine Einrichtung zur Applikationsüberwachung der Teilelektroden der Neutralelektrode umfaßt. Die Erfindung bezieht sich ferner auf ein Verfahren zur Wiederverwendung einer solchen Neutralelektrode.

Eine mehrteilige neutrale Elektrode mit drei flächenhaften Teilelektroden, die neben- oder übereinander angeordnet sind, ist aus der EP-A-0 383 982 bekannt.

Aus der DE-A-20 21 295 ist eine inaktive (neutrale) mehrteilige Elektrode zur Verwendung an elektrochirurgischen Geräten bekannt, bestehend aus einem nichtleitenden Trägermaterial, auf welchem ein Paar flexibler Elektroden befestigt ist, wobei zum Anheften der Elektroden am menschlichen Körper ein Haftmittel (Klebstoff) auf dem die Elektroden umgebenen Tägermaterial vorgesehen ist. Damit ist das überstehende, die Elektroden tragende Trägermaterial gegen den Körper des Patienten klebbar. Vor dem Gebrauch der nur einmal verwendbaren Neutralelektrode ist auf der zu applizierenden Fläche eine abziehbare Schutzfolie vorgesehen.

In der DE-A-35 44 443 ist eine Schaltungsanordnung zur Überwachung der neutralen Elektrode eines HF-Chirurgiegerätes auf flächiges Anliegen der Elektrode am Körper des Patienten beschrieben. Durch Fehlapplikation oder Ablösen der neutralen Rückstrom-Elektrode kann es in der HF-Chirurgie zu unerwünschten Verbrennungen des Patienten kommen. Durch mehrteilige neutrale Elektroden und eine Überwachungseinrichtung soll das Ablösen der Elektrode rechtzeitig erkannt und dem Arzt signalisiert werden. Es ist bereits bekannt, zur Erhöhung der Patientensicherheit die neutrale Elektrode in mehrere Teilelektroden zu unterteilen. Bei einer solchen Einrichtung wird ein Hilfs- oder Meßstrom von einem Meßstromgeber zur einen Teilelektrode, von dort über den Patienten zur anderen Teilelektrode und von dieser schließlich zurück zum Meßstromgeber geleitet und überwacht. Wenn dieser Stromkreis geschlossen ist, dann ist sichergestellt, daß jede Teilelektrode praktisch ganzflächig am Patienten anliegt, so daß der eigentliche HF-Arbeitsstrom appliziert werden kann. Eine solche unterteilte neutrale Elektrode ermöglicht somit die Feststellung, ob diese richtig angebracht ist.

Für diese bekannten mehrteiligen Elektroden ist jedoch eine Anordnung der Teilelektroden gewählt, die ein teilweises Ablösen von Teilelektroden ermöglicht, ohne daß dadurch die Überwachungsschaltung anspricht und Ablösealarm auslöst. Dieser Alarm erfolgt aufgrund der bekannten Elektrodenausbildung oftmals zu spät, da die Überwachungseinrichtung auf das Ablösen der Teilelektroden je nach Ablöserichtung der Elektrode unterschiedlich anspricht. Die bekannten mehrteiligen Elektroden sind so angeordnet bzw. ausgebildet, daß sie nach einer Ablöse-Symmetrie-Linie vom Körper des Patienten teilweise abgezogen werden können, ohne daß die beispielsweise Impedanzmessungen zwischen den auf dem Körper des Patienten aufliegenden Teilflächen der mehrteiligen Elektrode ausführende Überwachungseinrichtung rechtzeitig anspricht und Alarm auslöst.

Wegen der von Patient zu Patient unterschiedlichen Leitwerte des Patientengewebes liefert eine Widerstands- oder Kapazitätsmessung zwischen zwei Teilelektroden einer neutralen HF-Chirurgieelektrode noch kein ausreichend sicheres Ergebnis dafür, ob die Kontaktfläche zwischen der Elektrode und dem Patienten ausreichend ist, um Verbrennungen des Patienten zu vermeiden. Weist beispielsweise das Gewebe eines Patienten nur ein Drittel des Gewebewiderstandes eines anderen Patienten auf, so kann bei diesem niedrigen Gewebewiderstand etwa ein Drittel der Elektrodenfläche ausreichend sein, um einen voreingestellten Grenzwert für die Messung einzuhalten. Aus diesem Grunde ist es sinnvoll, mit Elektroden zu arbeiten, die mehrere Teilflächen aufweisen und bei denen durch eine Symmetriemessung von Teilströmen auf den gleichmäßigen Kontakt mit dem Patienten geschlossen wird. Für die Symmetriemessung kann dabei ein Hilfsstrom oder der HF-Chirurgiestrom selbst verwendet werden.

Wegen der Patienten- und Anwendersicherheit werden diese bekannten Elektroden bei der Anwendung in der HF-Chirurgie mit einer selbstklebenden applizierenden Fläche ausgebildet, die durch eine Schutzfolie abgedeckt ist. Zur Operation wird die Schutzfolie abgezogen und die Elektrode gegen den Patientenkörper geklebt. Nach der Operation wird die Elektrode vom Kabelanschluß zum HF-Chirurgiegerät getrennt und vom Patientenkörper abgezogen. Alle diese Neutralelektroden können nicht wiederverwendet werden, was erhebliche Kosten- und Umweltprobleme verursacht.

Die bekannten, in der Regel einteilig ausgebildeten, billigeren Einfach-Elektroden genügen aber den Sicherheitsansprüchen bei der HF-Chirurgie nicht bzw. sind für den Einsatz einer Meßeinrichtung für die Applikationsüberwachung nicht geeignet. Auch führt die Einmalverwendung dieser Wegwerfelektroden ebenfalls zur Umweltbelastung.

Da in der HF-Chirurgie die erforderliche Applikationsüberwachung der Teilelektroden ohne Fehlalarmierung ständig einen weitgehend vollflächigen Kontakt der Neutralelektrode mit dem Patientenkörper voraussetzt, ist eine Verwendung der bekannten Elektroden mit selbstklebender Oberfläche erforderlich, um den vollständigen Kontakt zu gewährleisten. Aus Hygienegründen können diese Elektroden nicht wiederverwendet werden.

Durch den Klebevorgang wird bei korrekter Elektrodenapplikation der vollständige Kontakt der gesamten aktiven (leitfähigen) Elektrodenoberfläche mit dem Patienten sichergestellt. Der Überwachungsschaltkreis des HF-Chirurgiegerätes erkennt korrekte Applikation und gibt den Hochfrequenzstrom zur Anwendung frei. Bei fehlerhafter Applikation, wenn die aktive Elektrodenfläche nicht ausreichend mit dem Patienten in Verbindung steht, durch welche Umstände dies auch immer verursacht wurde, oder bei komplett fehlender Neutralelektrode, erzeugt das HF-Gerät einen Alarm und blockiert gegebenenfalls die HF-Stromabgabe. Diese Funktion ist mit einer Neutralelektrode mit geteilter aktiver Elektrodenfläche, die nicht in selbstklebender Ausführung gefertigt ist, sondern in bekannter Ausführung (EP-A-0 223 340) als wiederverwendbare Einflächenelektrode ohne Applikationsüberwachungsmöglichkeit verwendet wird, nicht erreichbar. Derartige nicht selbstklebende Elektroden werden mit Bändern am Patienten befestigt, wodurch aber der vollständige Kontakt der leitfähigen Elektrodenfläche mit dem Patienten nicht sicherzustellen ist. Bei Neutralelektroden ohne Selbstklebefunktion entstehen zwangsläufig bereits beim Applizieren der Neutralelektrode Luftblasen zwischen der Neutralelektrode und dem Patienten, hervorgerufen durch Körperunebenheiten, Knochenvorsprünge, Ablöseerscheinungen durch Patientenbewegung usw. Diese Ablösungen verursachen Fehlalarme des HF-Gerätes, da bei Verwendung einer solchen Neutralelektrode die Alarmauslösetoleranz nicht definiert festgelegt werden kann. Einerseits müssen Verbrennungen ohne rechtzeitige Alarmierung verhindert werden, andererseits darf aber auch noch kein Fehlalarm erzeugt werden, wenn die nicht in Verbindung mit dem Patienten stehende Elektrodenfläche noch keine kritischen Werte erreicht hat.

Unter Berücksichtigung der vorbeschriebenen Problematik ist es die Aufgabe der Erfindung, eine wiederverwendbare Neutralelektrode der eingangs genannten Art zu schaffen, die in Verbindung mit dem HF-Chirurgiegerät die erforderliche Applikationsüberwachung ermöglicht und die Kostensowie Umweltbelastung besonders berücksichtigt.

Nach der Erfindung wird diese Aufgabe bei einer mehrteiligen Neutralelektrode der eingangs genannten Art dadurch gelöst, daß vor einer Operation auf die gegen den Patientenkörper zu applizierende Fläche der Neutralelektrode ein beidseitig haftendes Beschichtungsmittel aufbringbar ist, das eine erste Klebeseite zwischen dem Beschichtungsmittel und der Neutralelektrode und eine zweite Klebeseite zwischen dem Beschichtungsmittel und dem Patientenkörper aufweist und daß das Beschichtungsmittel nach der Operation von der wiederverwendbaren Neutralelektrode entfernbar ist.

Vorteilhafte Ausgestaltungen der Neutralelektrode nach der Erfindung sind in den Patentansprüchen 2 bis 10 angegeben.

Die Erfindung löst die gestellte Aufgabe ferner durch das in den Patentansprüchen 11 und 12 angegebene Verfahren zur Wiederverwendung einer mehrteiligen Neutralelektrode.

Die Neutralelektrode nach der Erfindung ist unter Verwendung von Metallelektroden, leitfähigem Gummi, leitfähigem Kunststoff usw. herstellbar, jedoch mit geteilten leitfähigen Teilelektrodenflächen und elektrischer Isolierung zwischen den einzelnen Teilelektroden. Auf diese wiederverwendbare Neutralelektrode wird vor der Operation ein zu der Elektrodenausführung passender Klebefilm aufgebracht, der nach Operationsende wieder abgezogen und weggeworfen wird. Die Neutralelektrode selbst, gegebenenfalls mit Anschlußkabel, kann wiederverwendet werden. Der Klebefilm kann je nach dem vom HF-Gerät benötigten Überwachungs- und Neutralelektrodenfunktionsprinzip elektrisch leitfähig oder auch isolierend sein. Durch diese Anordnung mit wiederverwendbarer Neutralelektrode und wegwerfbarem Klebefilm entstehen nur noch minimale Abfallmengen bei gleichzeitig geringen Betriebskosten.

Das nach der Erfindung vorgesehene Beschichtungsmittel bzw. der dabei zu verwendende Klebefilm kann sich insbesondere durch folgende Eigenschaften auszeichnen:
- er paßt in Form und Größe zu der zugehörigen Neutralelektrode,
- er ist ausreichend elektrisch leitfähig, wenn die Überwachungsschaltung des HF-Gerätes eine Impedanzmessung durchführt und die Neutralelektrode galvanisch mit dem Patienten verbunden sein muß,
- bei kapazitiver Messung des HF-Gerätes und kapazitiv wirkender Neutralelektrode ist auch eine nicht leitfähige Ausführung des Klebefilms ausreichend,
- die Klebekraft des Klebefilms ist so gewählt, daß die Haftung zur Neutralelektrode hin etwas größer ist als zum Patienten, damit der Klebefilm beim Abziehen der Neutralelektrode möglichst an dieser haften bleibt,
- der Klebefilm ist durch einen mechanischen Träger verstärkbar, so daß der Klebefilm in einem Stück nach der Operation abgezogen werden kann; dies ist erreichbar durch z.B. den Einsatz einer durchlöcherten Trägerfolie oder eines netz- bzw. siebartigen Materials aus z.B. Isolierstoff. Das Verhältnis von Öffnungen zu Stegen in diesem Trägermaterial wird dabei so gewählt, daß eine ausreichende Leitfähigkeit bei Elektroden für HF-Geräte mit Impedanzmessung gewährleistet ist.
- Für HF-Geräte mit Impedanzmessung ist die Leitfähigkeit des vollflächig angeordneten Klebefilmes so gewählt, daß einerseits die leitfähigen Teilelektroden der Neutralelektrode noch ausreichende galvanische Verbindung zum Patienten haben, andererseits die Applikationsüberwachungsschaltung noch arbeiten kann;
- bei kritischen Applikationsüberwachungsschaltungen kann der leitfähige Klebefilm geometrisch auf die Teilsegmente der Elektrode beschränkt bleiben. Zwischen den Teilsegmenten ist dann der leitfähige Klebefilm wegzulassen oder durch einen nicht leitfähigen Kleber zu ersetzen.

Weitere Vorteile und Einzelheiten der Erfindung ergeben sich aus der nachfolgenden Beschreibung von Ausführungsbeispielen und anhand der Zeichnung.

Es zeigen:
Figur 1 eine dreiteilige neutrale Elektrode für ein HF-Chirurgiegerät in Draufsicht auf ihre die Teilelektroden tragende, aktive und zu applizierende Fläche,
Figur 2 einen Querschnitt durch eine erfindungsgemäße, wiederverwendbare Neutralelektrode mit beispielsweise drei Teilelektroden,
Figur 3 einen Querschnitt gemäß Figur 2, wobei das Abziehen eines einmalverwendbaren Beschichtungsmittels von der wiederverwendbaren Neutralelektrode demonstriert ist,
Figur 4 in schematischer Darstellung einen Flächenabschnitt eines einmalverwendbaren Beschichtungsmittels unter Verwendung eines Trägernetzes und
Figur 5 einen Schnitt durch ein Beschichtungsmittel, bestehend aus einem beidseitig Klebefilme aufweisenden Trägernetz und mit beidseitig vorgesehenen, abziehbaren Schutzfolien.

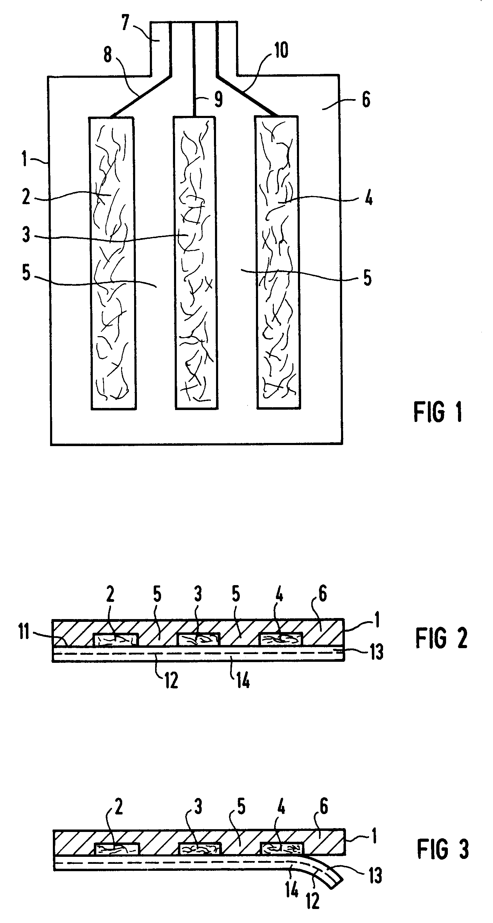

Nach dem Ausführungsbeispiel gemäß Figur 1 umfaßt die wiederverwendbare neutrale Elektrode 1 für ein HF-Chirurgiegerät drei flächenhafte Teilelektroden 2, 3 und 4, die z.B. nebeneinander auf einem elastischen Träger 6 so angeordnet sind, daß die Teilelektroden durch Isolierstreifen 5 oder Streifen geringer elektrischer Leitfähigkeit voneinander getrennt sind. Die Teilelektroden bestehen jeweils z.B. aus einer elektrisch leitenden Folie, z.B. einer Metallfolie oder einem Metallnetz, und sind an dem Träger 6 befestigt. Der Träger 6 steht am Rande über die Teilelektroden über und bildet einen Leitungsanschluß 7, an dem die Verbindungsleitungen 8, 9, 10 der drei Teilelektroden zusammengeführt sind. Die Neutralelektrode ist über ihren Leitungsanschluß 7 und über einen nicht gezeichneten Stecker mit einem Anschlußkabel des HF-Chirurgiegerätes verbindbar.

Gemäß den Figuren 2, 3 ist auf die die Teilelektroden 2, 3, 4 aufweisende aktive Fläche der Neutralelektrode 1, welche gegen einen Patientenkörper die zu applizierende Fläche 11 bildet, ein Beschichtungsmittel 12 bis 14 aufbringbar. Dieses Beschichtungsmittel umfaßt eine Zwischenschicht oder Trägerfolie 12 od.dgl., welche beidseitig Klebefilme 13, 14 aufweist. Das nur jeweils einmalverwenbare Beschichtungsmittel trägt zur Aufbewahrung an den Klebefilmen 13, 14 Schutzfolien 15 bzw. 16. Zur Vorbereitung einer Operation wird nach Abnahme der Schutzfolie 15 das Beschichtungsmittel mit seiner Klebefläche 13 gegen die zu applizierende Fläche 11 der wiederverwendbaren Neutralelektrode 1 geklebt. Nach Abziehen der Schutzfolie 16 kann die Neutralelektrode mit der Klebefläche 14 des Beschichtungsmittels 12 festklebend am Patientenkörper angebracht werden. Nach Beendigung der Operation wird die Neutralelektrode vom HF-Chirurgiegerät gelöst, vom Patientenkörper abgezogen und daraufhin das Beschichtungsmittel von der Neutralelektrode getrennt, z.B. ebenfalls durch Abziehen. Das Beschichtungsmittel wird weggeworfen und die Neutralelektrode ist nach entsprechender Reinigung in Verbindung mit einem Ersatz-Beschichtungsmittel erneut verwendbar.

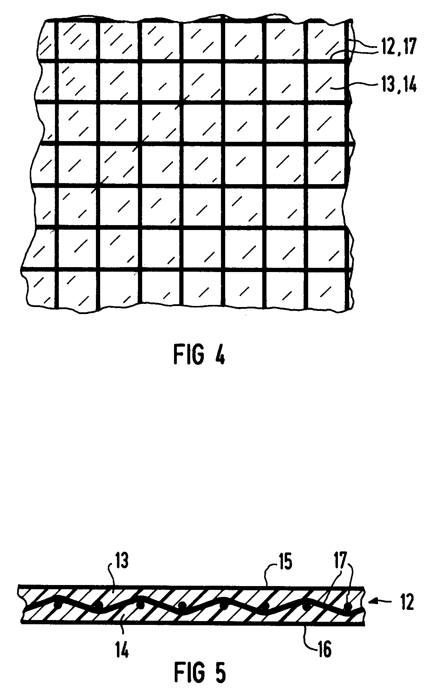

Gemäß der Ausführung nach den Figuren 4, 5 besteht das Beschichtungsmittel aus einem beidseitig Klebefilme 13, 14 aufweisenden Trägernetz 17, wobei die Klebefilme durch abziehbare Schutzfolien 15 bzw. 16 abgedeckt sind.

Als Träger für den Klebefilm kann eine gelochte Folie oder ein netzartiges Material mit feinen Fäden zur Verwendung kommen. Dadurch wird die zum Aufbringen auf die Neutralelektrode und zum Abziehen erforderliche Handhabbarkeit erreicht. Die Löcher bzw. Öffnungen sind nur für HF-Geräte notwendig, die galvanische Verbindung der Neutralelektrode zum Patienten erfordern sowie für Geräte, deren Überwachungssystem Impedanzmessungen durchführt, wenn das netzartige Trägermaterial aus Isolierstoff besteht. Ein elektrisch leitfähiges Trägermaterial erfordert keine Löcher bzw. Öffnungen. Bei kapazitiv arbeitenden Geräten können die Löcher bzw. Öffnungen jedenfalls wegfallen. Die gesamte Klebefolie mit integriertem Trägermaterial braucht nicht dicker als etwa 0,5 mm zu sein, wodurch nur geringe Abfallmengen entstehen. Zur leichteren Abziehbarkeit kann der Klebefilm eine über die Neutralelektrodenfläche überstehende Abzugslasche aufweisen.

## Patentansprüche

1. Mehrteilige Neutralelektrode (1) für ein Hochfrequenz-Chirurgiegerät, bei der Teilelektroden (2, 3, 4) durch Isolierstreifen (5) getrennt auf einem isolierenden, elastischen Träger (6) angeordnet sind, wobei die Teilelektroden über einen am Träger vorgesehenen elektrischen Leitungsanschluß (7) und ein Elektrodenkabel an das HF-Chirurgiegerät anschließbar sind, das eine Einrichtung zur Applikationsüberwachung der Teilelektroden der Neutralelektrode umfaßt,
**dadurch gekennzeichnet,**
daß vor einer Operation auf die gegen den Patientenkörper zu applizierende Fläche (11) der Neutralelektrode (1) ein beidseitig haftendes Beschichtungsmittel (12 bis 14) aufbringbar ist, das eine erste Klebeseite (13) zwischen dem Beschichtungsmittel und der Neutralelektrode und eine zweite Klebeseite (14) zwischen dem Beschichtungsmittel und dem Patientenkörper aufweist und daß das Beschichtungsmittel nach der Operation von der wiederverwendbaren Neutralelektrode (1) entfernbar ist.

2. Neutralelektrode nach Anspruch 1,
**dadurch gekennzeichnet,**
daß das Beschichtungsmittel aus einer beidseitig mit einem Klebefilm (13, 14) versehenen Zwischenschicht (12) besteht und auf beiden Außenflächen abziehbare Schutzfolien (15, 16) trägt.

3. Neutralelektrode nach Anspruch 2,
**dadurch gekennzeichnet,**
daß der Klebefilm (13, 14) leitfähig ist.

4. Neutralelektrode nach Anspruch 2,
**dadurch gekennzeichnet,**
daß die Zwischenschicht (12) aus einem Trägermaterial für die Klebefilme (13, 14) besteht, das für die Funktion der Überwachungseinrichtung des HF-Chirurgiegerätes eine ausreichende elektrische Leitfähigkeit aufweist oder aus einem Isoliermaterial mit Öffnungen besteht und die Neutralelektrode (1 bis 4) galvanisch mit dem Patientenkörper kontaktiert.

5. Neutralelektrode nach Anspruch 2,
**dadurch gekennzeichnet,**
daß die Zwischenschicht (12) aus einer Trägerfolie besteht, die beidseitig einen Klebefilm (13, 14) und abziehbare Schutzfolien (15, 16) aufweist.

6. Neutralelektrode nach Anspruch 5,
**dadurch gekennzeichnet,**
daß eine leitfähige Trägerfolie (12) oder ein Isoliermaterial mit Öffnungen beidseitig mit leitfähigem Klebstoff (13, 14) beschichtet ist.

7. Neutralelektrode nach Anspruch 5 oder 6,
**dadurch gekennzeichnet,**
daß die leitfähige Trägerfolie aus einer Matte oder Folie mit eingebetteten epitropischen Fasern, feinen Kohlenstoffpartikeln, einer dünnen Metallfolie od.dgl. besteht.

8. Neutralelektrode nach einem der Ansprüche 2 bis 7,
**dadurch gekennzeichnet,**
daß die Adhäsion der ersten mit der zu applizierenden Fläche (11) der Neutralelektrode zu verbindenden Klebeseite (13) der Zwischenschicht (12) größer als die Adhäsion der zweiten, am Patientenkörper anzubringenden Klebeseite (14) ist.

9. Neutralelektrode nach den Ansprüchen 1 bis 3,
**dadurch gekennzeichnet,**
daß das Beschichtungsmittel (12) auf der Verbindungsseite der zu applizierenden Fläche (11) der Neutralelektrode (1) auf den den Isolierstreifen (5) zugewandten Flächenstreifen mit einem nicht leitfähigen Kleber beschichtet ist.

10. Neutralelektrode nach einem der Ansprüche 2 bis 9,
**dadurch gekennzeichnet,**
daß die Zwischenschicht (12) aus einem Trägernetz (17), einer gelochten Trägerfolie, einer Zwischenschicht aus porösem Material, einer Isolierstoffmatte aus netz- oder siebförmigem Material od.dgl. besteht, wobei das Verhältnis der Öffnungen bzw. Durchbrüche zu den Stegen des Trägermaterials so ausgelegt ist, daß eine ausreichende Leitfähigkeit zwischen den Teilelektroden (2, 3, 4) der Neutralelektrode (1) über das Beschichtungsmittel (12 bis 14) zum Patientenkörper für die Überwachungs-Meßeinrichtung des HF-Chirurgiegerätes gewährleistet ist.

11. Verfahren zur Wiederverwendung einer mehrteiligen Neutralelektrode nach den Ansprüchen 1 bis 10,
**dadurch gekennzeichnet,**
daß zum Gebrauch der Neutralelektrode auf deren gegen den Patientenkörper zu applizierenden Fläche ein Beschichtungsmittel aufgebracht wird, wobei eine Schutzfolie vom Beschichtungsmittel abgezogen wird und das Beschichtungsmittel auf die Neutralelektrode klebbar ist, wobei eine weitere Schutzfolie vom Beschichtungsmittel abgezogen wird und die Neutralelektrode unter Zwischenlage des Beschichtungsmittels mit deren selbstklebenden Oberfläche gegen den Patientenkörper klebbar ist, wobei nach der Operation die Neutralelektrode mit dem Beschichtungsmittel vom Patientenkörper lösbar ist und wobei daraufhin das Beschichtungsmittel von der wiederverwendbaren Neutralelektrode abziehbar ist.

12. Verfahren nach Anspruch 11,
**dadurch gekennzeichnet,**
daß die wiederverwendbare, Teilelektroden aufweisende Neutralelektrode mit einem einmalverwendbaren, abziehbaren Klebefilm versehen ist, der nach jedem Gebrauch der Neutralelektrode durch einen Ersatz-Klebefilmstreifen ersetzbar ist.
